Europäisches Patentamt

European Patent Office

Office européen des brevets

(19)

(11) Veröffentlichungsnummer: **0 277 280**
**B1**

(12) **EUROPÄISCHE PATENTSCHRIFT**

(45) Veröffentlichungstag der Patentschrift: 30.01.91

(21) Anmeldenummer: 87115633.7

(22) Anmeldetag: 24.10.87

(51) Int. Cl.⁵: **A 61 F 2/08**

(54) Kreuzbandersatz für ein Kniegelenk.

(30) Priorität: 07.01.87 CH 29/87

(43) Veröffentlichungstag der Anmeldung:
10.08.88 Patentblatt 88/32

(45) Bekanntmachung des Hinweises auf die
Patenterteilung:
30.01.91 Patentblatt 91/05

(84) Benannte Vertragsstaaten:
AT DE FR GB IT

(56) Entgegenhaltungen:
EP-A-0 051 954
EP-A-0 201 667

AMERICAN SOCIETY FOR ARTIFICIAL
INTERNAL ORGANS, Band 31, 1.-3. Mai 1985,
Seiten 305-307, Atlanta, Georgia, US; P. REY et
al.: "New fiber for ligament and tendon
prosthesis"

(73) Patentinhaber: GEBRÜDER SULZER
AKTIENGESELLSCHAFT
Zürcherstrasse 9
CH-8401 Winterthur (CH)

(73) Patentinhaber: Protek AG
Stadtbachstrasse 64
CH-3001 Bern (CH)

(72) Erfinder: Mansat, Christian, Dr.-med.
Route de Gaure
F-31130 Balma (FR)

(74) Vertreter: Sparing Röhl Henseler Patentanwälte
European Patent Attorneys
Rethelstrasse 123
D-4000 Düsseldorf 1 (DE)

Courier Press, Leamington Spa, England.

## Beschreibung

Die Erfindung betrifft einen Kreuzbandersatz für ein Kniegelenk, der auf mindestens einem Teil seiner Länge einen Strang für das vordere und einen Strang für das hintere Kreuzband bildet, wobei die Stränge aus einer Vielzahl konzentrischer, textiler Schläuche aufgebaut sind, die als geschlossene Ringe "jahresringartig" übereinander gelagert sind, wobei ferner jeder Strang mindestens einen Abschnitt relativ grosser Längsdehnbarkeit und relativ grosser Biegsamkeit aufweist.

Ein künstliches Band der vorstehend genannten Art ist aus der EP 201 667 bekannt. Wird dieses künstliche Band als Kreuzbandersatz im Kniegelenk eingesetzt und werden gleichzeitig beide natürlichen Kreuzbänder ersetzt, so ist es bisher üblich, je eines dieser bekannten Konstruktionen als vorderes und als hinteres Kreuzband getrennt und "nebeneinander liegend" einzusetzen. In der Praxis hat sich nun gezeigt, dass diese beiden künstlichen Kreuzbänder bei ihren Relativbewegungen zueinander einen relativ grossen Abrieb und Verschleiss erleiden, was nicht toleriert werden kann.

Aufgabe der Erfindung ist es, Reibung und Abrieb bei einem künstlichen Kreuzband-Ersatz für Fälle, bei denen gleichzeitig das vordere und das hinteres Kreuzband durch eine Prothese ersetzt werden, möglichst weitgehend zu verringern.

Diese Aufgabe wird dadurch gelöst, dass die beiden Stränge für das vordere und das hintere Kreuzband sich in dem flexiblen Abschnitt gegenseitig durchsetzen; im einfachsten Fall wird dabei ein Strang — beispielsweise mit einem ahlenartigen Instrument — durch den flexiblen Bereich eines zweiten oder des gleichen Bandes gezogen.

Bei zwei sich derart durchsetzenden künstlichen Kreuzbänder ist die Relativbewegung gegeneinander — und damit auch Reibung und Abrieb — sehr stark vermindert, da sich die Bänder in erster Linie aufgrund ihrer grossen Längsdehnbarkeit ausdehen, ehe sie aufeinander zu "gleiten" beginnen.

Werden zwei verschiedene künstliche Bänder für den Ersatz eines vorderen und eines hinteren Kreuzbandes verwendet, so sind vier Stellen für die Fixierung am Knochen notwendig. Wenn jedoch die Stränge für das vordere und für das hintere Kreuzband aus dem selben Band bestehen, das zwischen beiden Strängen eine Schlaufe bildet, lässt sich die Anzahl der Verankerungsstellen am Knochen auf eine oder zwei verringern, wie noch beschrieben wird.

Im folgenden wird die Erfindung anhand zweier Ausführungsbeispiele im Zusammenhang mit der Zeichnung näher erläutert.

Fig. 1 ist eine Darstellung des neuen Kreuzbandersatzes, der aus zwei getrennten Strängen besteht;

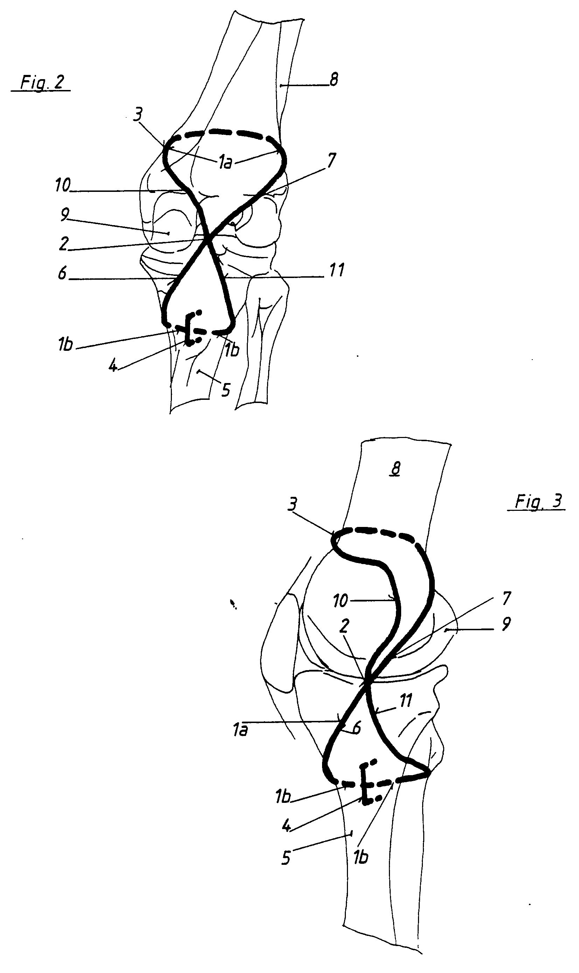

Fig. 2 gibt schematisch die Ansicht eines Kniegelenks von posterior her wieder, in das ein Ersatz beider Kreuzbänder implantiert ist, der aus einem einzigen Band gefertigt ist;

Fig. 3 ist eine Ansicht von Fig. 2 von links, d.h. von medial her gesehen.

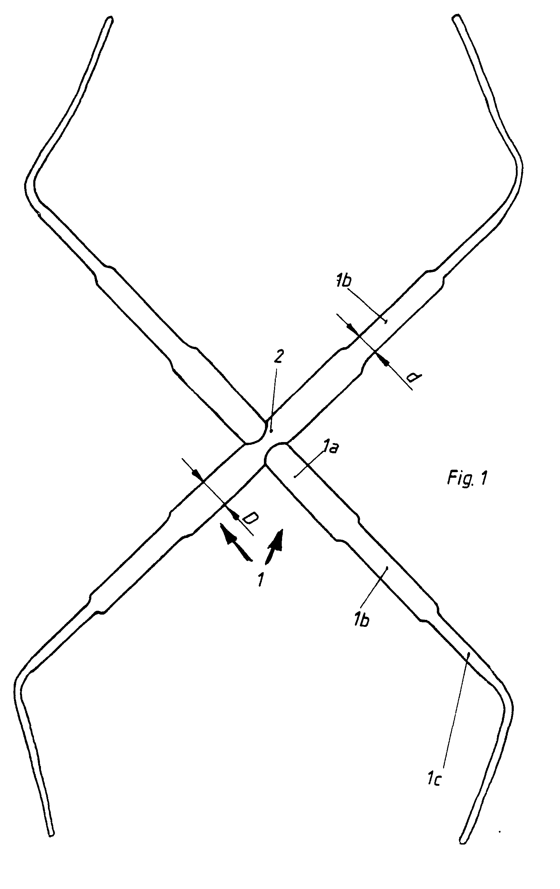

Wie das erwähnte, bekannte Band hat jeder der gleichartigen Stränge 1 nach Fig. 1 einen flexiblen zentralen Bereich 1a, dessen Durchmesser mit D bezeichnet ist; an den Bereich 1a schliessen links und rechts Bereiche 1b mit einem Durchmesser d, die eine relativ geringe Flexibilität haben. Bei dem etwa in natürlicher Grösse wiedergegebenen Band 1 nach Fig. 1 betragen der Durchmesser im flexiblen Bereich beispielsweise etwa 12 mm und derjenige im steifen Bereich etwa 7 mm. Jeder Strang 1 ist in bekannter Weise kernlos aus einer Vielzahl von konzentrischen Lagen aufgebaut; eine vorteilhafte Ausführungsform erhält man dabei, wenn der Strang 1 aus geflochtenen Schläuchen besteht, deren Flechtenwinkel gegen die Achsrichtung in den Bereichen unterschiedlicher Flexibilität verschieden, jedoch innerhalb eines Bereiches für alle Schläuche untereinander gleich sind. Neben Flechten können jedoch auch andere für die Verarbeitung von Fäden bekannte Verfahren, wie Weben, Stricken, Wirken usw. benutzt werden, die einen Mindestwert für die elastische Längsdehnbarkeit des Bandes zulassen.

Die Schläuche können aus multifilen oder monofilen Fäden hergestellt sein; weiterhin können als Fäden Naturfasern, z.B. Seide, oder Kunstfasern, beispielsweise Polyester, Polyäthylen oder auch bekannte resorbierbare Materialien verwendet werden. Zur Herstellung eines Stranges 1 werden eine Vielzahl von konzentrischen Schläuchen oder Lagen, beispielsweise 20 bis 30, übereinander "geschichtet".

Für die Herstellung von geflochtenen Schläuchen haben sich besonders gezwirnte Fäden aus einer Vielzahl, z.B. 60, Monofilamenten bewährt, die beispielsweise aus Polyester bestehen und Durchmesser von 0,01 bis 0,03 mm haben.

An den Enden sind einige wenige oder nur der äusserste Schlauch als dünne Einfädelabschnitte 1c ausgebildet, die unter Umständen durch eine Einlage oder ein Tränken in einer härtenden Masse versteift sind.

Die unterschiedliche Längsdehnbarkeit und die unterschiedliche Flexibilität der einzelnen Bereiche 1a und 1b werden beim Aufbau eines Stranges 1 aus geflochtenen Schläuchen durch unterschiedliche Flechtwinkel gegen die Bandachse erreicht. Dabei ist die Flexibilität umso grösser je flacher der Flechtwinkel gegen die Bandachse geneigt ist.

Erfindungsgemäss durchsetzen sich bei der Ausführungsform nach Fig. 1 die beiden Stränge 1 in ihrem flexiblen Bereich 1a in einem Kreuzungspunkt 2. Eine einfache Methode, ein beide Kreuzbänder ersetzendes künstliches Band zu erzeugen, besteht darin, einen der beiden Stränge 1 mit Hilfe einer Ahle durch den Bereich 1a eines anderen Stranges 1 zu ziehen. Die hohe Dehnbarkeit des Geflechtes im flexiblen Bereich 1a fixieren dabei die Stränge 1 am Kreuzungspunkt 2 gegenseitig, wodurch ein "Gleiten" und Reiben der Stränge 1 gegeneinander weitgehend verhindert wird.

Bei der zweiten Ausführungsform der Erfindung besteht das Implantat aus einem einzigen Strang 1, der eine Schlaufe 3 bildet und dessen Kreuzungspunkt 2 erst intraoperativ hergestellt wird.

Ausgehend von einer Agraffe 4, mit der ein wenig flexibler Bereich 1b am Tibiaknochen 5 verankert ist, durchsetzt der Strang 1 zunächst eine erste Knochenbohrung 6 im Tibiaknochen 5 von links unten nach rechts oben, um — nachdem er einen Ersatz eines der Kreuzbänder gebildet hat — in gleicher Richtung fortlaufend durch eine erste femurale Knochendurchführung 7 auf die laterale Aussenseite des Femurknochens 8 zu gelangen. Der Strang wird dann oberhalb der Kondylen 9 aussen anterior um den Femur 8 geschlungen und durch eine zweite Knochenbohrung 10 im Femur 8 geführt.

Zur Bildung des zweiten Kreuzbandes verläuft der Strang dann zwischen Femur 8 und Tibia 5, wobei — wie erwähnt — intraoperativ auf die schon beschriebene Weise der Kreuzungspunkt 2 gebildet wird. Durch eine weitere Durchführung 11 im Tibiaknochen 5 und mit einer teilweisen Umschlingung des Tibiaknochens 5 wird der Strang zur Agraffe 4 zurückgeführt; durch diese werden die beiden Enden 1b gemeinsam verankert.

Selbstverständlich ist es auch möglich, jeden Endbereich 1b mit einer eigenen Agraffe oder auf andere bekannte Weise zu fixieren. Weiterhin kann man auf eine der Femurdurchführungen 7 oder 10 verzichten und den Strang auf einem seiner Wege zur Schlaufe 3 hin an der Knochenoberfläche zwischen den Kondylen 9 hindurchführen.

## Patentansprüche

1. Kreuzbandersatz für ein Kniegelenk, der auf mindestens einem Teil seiner Länge einen Strang (1) für das vordere und einen Strange (1) für das hintere Kreuzband bildet, wobei die Sträng (1) auf einer Vielzahl konzentrischer, textiler Schläuche aufgebaut sind, die als geschlossene Ringe "jahresringartig" übereinander gelagert sind, wobei ferner jeder Strang mindestens einen Abschnitt (1a) relativ grosser Längsdehnbarkeit und relativ grosser Biegsamkeit aufweist, dadurch gekennzeichnet, dass die beiden Stränge (1) für das vordere und das hintere Kreuzband sich in dem flexiblen Abschnitt (1a) gegenseitig durchsetzen.

2. Kreuzbandersatz nach Anspruch 1, dadurch gekennzeichnet, dass die Stränge (1) für das vordere und für das hintere Kreuzband aus dem selben Band bestehen, das zwischen beiden Strängen (1) eine Schlaufe (3) bildet.

## Revendications

1. Ligament croisé de remplacement pour une articulation du genou, qui comprend sur au moins une partie de sa longueur, un cordon (1) pour le ligament croisé antérieur et un cordon (1) pour le ligament croisé postérieur, les cordons (1) étant constitués d'une pluralité de tubes textiles concentriques qui sont superposés en anneaux fermés, à la manière "d'anneaux de croissance annuelle", chaque cordon présentant en outre au moins une section (1a) d'extensibilité longitudinale relativement grande et de flexibilité relativement grande, caractérisé en ce que les deux cordons (1) pour le ligament croisé antérieur et le ligament croisé postérieur se traversent réciproquement dans la section flexible (1a).

2. Ligament croisé de remplacement selon la revendication 1, caractérisé en ce que les cordons (1) pour le ligament croisé antérieur et pour le ligament croisé postérieur sont constitués du même ligament qui forme une boucle (3) entre les deux cordons (1).

## Claims

1. A cruciate ligament prosthesis for a knee joint, the prosthesis forming over at least some of its length a strand (1) for the front cruciate ligament and a strand (1) for the rear cruciate ligament, the strands (1) being devised from a large number of concentric textile flexible tubular members disposed one above another as closed rings after the fashion of the growth rings of trees, each strand having at least one part (1a) of relatively great lengthwise stretch and relatively great flexibility, characterised in that the two strands (1) for the front and rear cruciate ligaments extend one through another in the flexible part (1a).

2. A prosthesis according to claim 1, characterised in that the strands (1) for the front and rear cruciate ligaments are made of the same band or strip or the like as forms a loop (3) between the two strands (1).

Fig. 1

Fig. 2

Fig. 3